# EUROPÄISCHE PATENTSCHRIFT

(11) **EP 2 901 961 B1**
(45) Veröffentlichungstag und Bekanntmachung des Hinweises auf die Patenterteilung: **14.03.2018**
(21) Anmeldenummer: 15152844.5
(22) Anmeldetag: 28.01.2015
(51) Int. Cl.: A61C 3/02

(54) **Dentalinstrument**
Dental instrument
Instrument dentaire

(30) Priorität: 03.02.2014 DE 102014201899
(43) Veröffentlichungstag der Anmeldung: 05.08.2015
(73) Patentinhaber: Gebr. Brasseler GmbH & Co. KG, 32657 Lemgo (DE)
(72) Erfinder: Hecke, Josef, 32694 Dörentrup (DE); Meier, Friedrich Wilhelm, 32825 Blomberg (DE); Niemeier, Markus, 32791 Lage (DE)
(74) Vertreter: Hoefer & Partner Patentanwälte mbB

(56) Entgegenhaltungen:
- WO-A2-2004/021913
- CH-A5- 651 746
- DE-T2- 69 636 020
- DE-U1-202008 018 364
- US-B1- 6 299 445

## Beschreibung

Die Erfindung bezieht sich auf ein Instrument, insbesondere ein Dentalinstrument oder ein chirurgisches Instrument, gemäß den Merkmalen des Oberbegriffs des Anspruchs 1.

Im Einzelnen bezieht sich die Erfindung auf ein Instrument mit einem Schaft sowie mit einem an dem Schaft befestigten, im Wesentlichen rotationssymmetrischen Kopf, welcher mit mehreren Schneiden versehen ist. Das Instrument ist um eine zentrische Drehachse drehbar. Die Schneiden sind um den gesamten Umfang des Kopfes, bezogen auf die Drehachse, verteilt am Kopf angeordnet.

Aus dem Stand der Technik sind unterschiedlichste Ausgestaltungen derartiger Instrumente bekannt. Beispielsweise wird auf die DE 198 10 284 A1 oder die DE 197 34 016 A1 hingewiesen.

Insgesamt liegt der Ausgestaltung derartiger Instrumente stets die Thematik zugrunde, dass diese eine ausreichende Abtragsleistung oder Schneidleistung sowie eine befriedigende Standzeit aufweisen müssen. Weiterhin wird gefordert, dass derartige Instrumente vibrationsarm arbeiten.

Die bekannten Instrumente, welche auch als Fräser bezeichnet werden, weisen üblicherweise um den Umfang verteilt identische Schneiden mit identischen Schneidengeometrien und Teilungen auf. Zusätzlich zu einer ersten Verzahnung, welche durch erste Schneiden gebildet wird, kann auch eine zweite Verzahnung oder Nebenverzahnung ausgebildet werden, welche ebenfalls schneidend ist und deren Schneiden beispielsweise einen zu den Hauptschneiden entgegengesetzten Drall aufweisen.

Weiterhin ist es bekannt, die Grundverzahnung eines derartigen Instruments so auszubilden, dass diese im Umfang in mehrere Gruppen unterteilt ist. In den einzelnen Gruppen können unterschiedliche Verzahnungen vorgesehen sein, die jedoch innerhalb der Gruppe gleich sind. Hierdurch ergibt sich bei der Rotation des Instruments eine Abfolge der in Eingriff befindlichen Schneiden von einer ersten Gruppe zu einer zweiten Gruppen. Auch derartige Ausgestaltungen erfüllen nicht stets die Erwartungen hinsichtlich der Abtragsleistung und des vibrationsarmen Rundlaufs.

Die DE 20 2008 018364 U1 beschreibt einen Dentalfräser mit einem rotationssymmetrischen Kopf, auf dessen Außenumfang Schneiden angeordnet sind, welche sich in Längsrichtung des Kopfes erstrecken und zwei unterschiedliche Grundverzahnungen aufweisen. In jeder Gruppe der Grundverzahnungen sind gleiche Schneidengeometrien vorgesehen.

Aus der WO 2004/021913 A2 ist eine endodontische Feile bekannt, bei welcher ein sich verjüngender, kegelförmiger Kopf mit Umfangsschneiden versehen ist. Dabei sind in einer Radialschnittebene jeweils zwei gleiche Schneiden ausgebildet.

Die CH 651 746 A5 offenbart ein Dentalinstrument, mit jeweils gleichen Schneiden, die durch Spanbrechernuten unterbrochen sind.

Aus der DE 696 36 020 T2 ist ein Endodontie-Instrument vorbekannt, welches keinen rotationssymmetrischen Kopf aufweist, sondern durch eine Verdrallung eines Ausgangskörper gebildet wird.

Ein weiteres endodontisches Instrument zeigt die US 6 299 445 B1. Auch hierbei sind in den Radialschnitten zumindest zwei gleiche Schneiden vorgesehen.

Der Erfindung liegt die Aufgabe zugrunde, ein Instrument der eingangs genannten Art zu schaffen, welches sich durch eine hohe Abtragsleistung und eine hohe Standzeit sowie durch einen vibrationsarmen Lauf auszeichnet.

Erfindungsgemäß wird die Aufgabe durch die Merkmalskombination des Anspruchs 1 gelöst. Die Unteransprüche zeigen weitere vorteilhafte Ausgestaltung der Erfindung.

Erfindungsgemäß ist somit vorgesehen, dass die um den Umfang des Kopfes verteilten Schneiden so ausgebildet sind, dass jede Schneide zu den anderen Schneiden eine unterschiedliche Geometrie aufweist. Das Instrument weist somit an seinem Kopf keine Schneide auf, welche zu einer anderen Schneide gleich ist. Vielmehr sind sämtliche Schneiden unterschiedlich ausgebildet.

Erfindungsgemäß kann die unterschiedliche Ausbildung der Schneiden sich auf die Teilung (Teilungswinkel in Umfangsrichtung), den Keilwinkel, den Freiwinkel, den Spanwinkel und/oder die Schneidentiefe beziehen. Sämtliche weiteren Unterschiede der Schneiden sind erfindungsgemäß mit umfasst.

Das erfindungsgemäße Instrument ist bevorzugterweise so ausgebildet, dass die Schneiden alle auf einem gemeinsamen Drehkreis angeordnet sind, welcher seinen Mittelpunkt in der Drehachse des Instruments hat. Hierdurch wird ein vibrationsarmer, gleichmäßiger Rundlauf des Instruments sichergestellt.

Das erfindungsgemäße Instrument kann aus einem metallischen Werkstoff, aus Keramik oder aus Kunststoff gefertigt sein. Die Schneiden können durch spanende Bearbeitung, beispielsweise Fräsen oder Schleifen, erzeugt werden. Dabei können die Schneiden insbesondere mittels einer CNC-Maschine erzeugt werden.

Durch die unterschiedliche Geometrie der Schneiden erfolgt bei einer Rotation des Instruments eine stets zur vorangehenden Schneide geänderte Schneidgeometrie. Dies betrifft insbesondere, wie erwähnt, die Schneidenwinkel (Keilwinkel, Spanwinkel, Freiwinkel). Durch die unterschiedliche Ausbildung der einzelnen Schneiden ändert sich jedoch auch die Schneidentiefe, so dass auch ein unterschiedlicher Spanraum ausgebildet wird. Dies führt zu verbesserten Abtragsleistungen. Die sich ergebenden unterschiedlichen Belastungen auf die einzelnen Schneiden resultieren in einer langen Standzeit des Instruments.

Erfindungsgemäß ist es möglich, an dem Kopf Schneiden gemäß der oben beschriebenen erfindungsgemäßen Lösung als Hauptverzahnung anzubringen und zusätzlich Schneiden einer Nebenverzahnung auszubilden. Diese kann beispielsweise eine andere Steigung oder einen anderen Drall aufweisen, als die Schneiden der Hauptverzahnung. Dabei ist es insbesondere vorteilhaft, wenn auch die Schneiden der Nebenverzahnung zueinander unterschiedlich ausgebildet sind. Sie können jedoch auch zueinander gleich ausgebildet sein.

Bevorzugterweise erstrecken sich die erfindungsgemäßen Schneiden im Wesentlichen über die gesamte axiale Länge des Kopfes. Der Kopf des Instruments kann erfindungsgemäß übliche Formen aufweisen, beispielsweise zylindrisch, konisch oder in balligen oder abgerundeten Formen, beispielsweise in Form einer Birne, einer Flamme oder einer Knospe. Insofern können sämtliche aus dem Stand der Technik bekannten Kopfformen mit der erfindungsgemäßen Verzahnung versehen werden.

Das erfindungsgemäße Instrument kann so ausgebildet sein, dass sich die Schneiden der Hauptverzahnung geradlinig oder gedrallt längs des Kopfes erstrecken. Die Schneiden können so ausgebildet sein, dass sie stirnseitig zur Mitte des Instruments (zur Drehachse) verlaufend ausgebildet sind. Es ist jedoch auch möglich, die Schneiden so auszugestalten, dass sie stirnseitig zur Ausbildung zumindest einer Querschneide zusammenlaufen. Auch die Unterteilung der Gruppen in Umfangsrichtung in einzelne Abschnitte oder Gruppen ist möglich. Diese Gruppen können sich beispielsweise hinsichtlich der Steigung oder des Dralls unterscheiden. Die Ausgestaltung der Schneiden in Gruppen kann auch zu der erwähnten Ausbildung zumindest einer Querschneide am vorderen Endbereich des Instruments dienen. Es ist auch möglich, die Schneiden in zwei oder vier Gruppen anzuordnen, welche hinsichtlich einer die Drehachse umfassenden Mittelebene gespiegelt oder symmetrisch ausgebildet sind. Es sind dann in jeder Gruppe unterschiedliche Schneiden oder Zähne ausgebildet.

Bei allen beschriebenen Ausgestaltungsvarianten ist erfindungsgemäß vorgesehen, dass sämtliche Schneiden zueinander unterschiedlich ausgebildet sind. Es sind am Kopf somit alle Schneiden voneinander verschieden, es gibt keine zwei identischen oder gleichen Schneiden.

In einer weiteren, besonders günstigen Ausgestaltung der Erfindung ist vorgesehen, dass die Schneidengeometrien zufällig, ggf. unter Zuhilfenahme eines Zufallsgenerators, definiert werden. Die Schneiden werden somit hinsichtlich ihrer Ausgestaltung (Teilung, Keilwinkel, Freiwinkel, Spanwinkel und/oder Schneidentiefe) oder hinsichtlich weiterer Parameter zufallsmäßig ausgestaltet. Dies bedeutet, dass kein Instrument dem anderen gleicht, dass sämtliche Instrumente jedoch dem selben Grundkonzept folgen.

Im Folgenden wird die Erfindung anhand eines Ausführungsbeispiels in Verbindung mit der Zeichnung beschrieben. Dabei zeigt:
- Fig. 1: eine Seitenansicht eines Kopfes eines Instruments gemäß der Erfindung;
- Fig. 2: eine Schnittansicht eines Ausführungsbeispiels des erfindungsgemäßen Instruments;
- Fig. 3: eine stirnseitige Draufsicht auf das in Fig. 2 gezeigte Instrument; und
- Fig.4: eine Darstellung unterschiedlicher Kopfformen des erfindungsgemäßen Instruments.

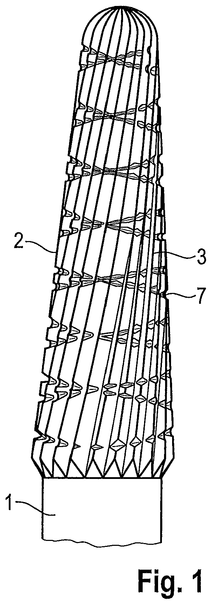

Die Fig. 1 zeigt ein Ausführungsbeispiel eines erfindungsgemäßen Instruments (Dentalinstrument oder chirurgisches Instrument) in der Teil-Seitenansicht, wobei ein Kopf 2 dargestellt ist, welcher konisch ausgebildet, an seiner Stirnseite abgerundet und an einem Schaft 1 befestigt ist. Der Kopf 2 ist mit gedrallten Schneiden 3 versehen, welche erfindungsgemäß ausgebildet sind und umfasst zusätzlich Nebenschneiden 7.

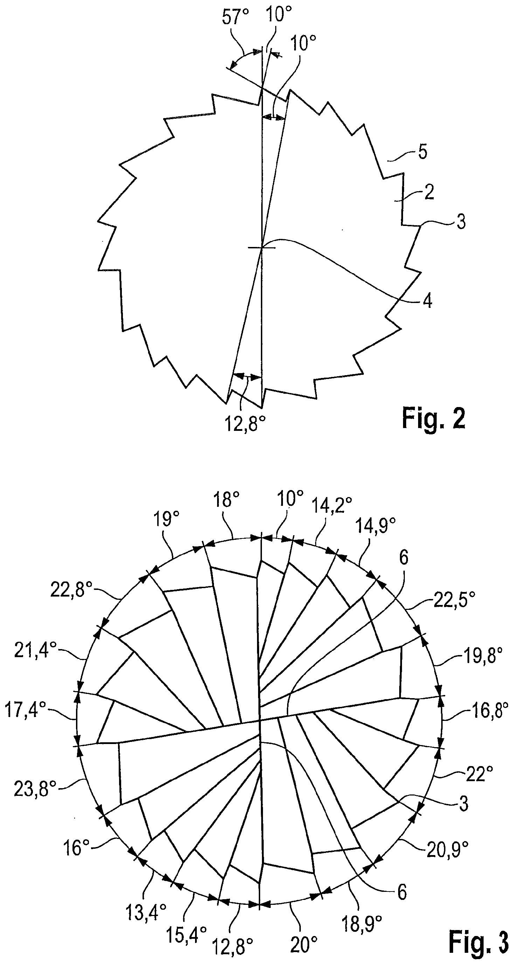

Die Fig. 2 und 3 zeigen eine Detailansicht eines Ausführungsbeispiels der erfindungsgemäßen Verzahnung. Dabei zeigt die Fig. 2 eine Schnittansicht in einer zu einer Drehachse 4 senkrechten Radialebene. Dabei ist ersichtlich, dass das Instrument um den Umfang verteilt eine Vielzahl von Schneiden 3 aufweist. Die Schneiden 3 können, wie in Fig. 1 gezeigt, gewendelt oder gedrallt ausgebildet sein. Es ist jedoch auch möglich, diese geradlinig auszubilden. Die Spitzen sämtlicher Schneiden liegen auf einem Drehkreis 5, so dass sich eine zur Drehachse 4 rotationssymmetrische Hüllkurve ergibt, wenn das Instrument um die Drehachse 4 in Rotation versetzt wird.

In der oberen Bildhälfte der Fig. 2 ist verdeutlicht, wie die Schneide ausgebildet ist. Sie weist einen Spanwinkel von 10° auf und ist zur nächsten, benachbarten Schneide mit einer Teilung von 10° ausgebildet. Die Darstellung der Fig. 2 zeigt weiterhin den zugeordneten Keilwinkel der Schneide. Dies ergibt sich aus dem Zahnwinkel von 57° zuzüglich des negativen Spanwinkels von 10°.

Die Fig. 2 zeigt in der unteren Bildhälfte eine zu der oben beschriebenen Schneide gegenüberliegende Schneide, welche mit einer Teilung von 12,8° zur nächstfolgenden Schneide versehen ist und sich somit von der in Fig. 2 oben liegenden Schneide unterscheidet.

Die Fig. 3 zeigt eine stirnseitige Ansicht des in Fig. 2 gezeigten Instruments. Dabei ist eine Anzahl von Schneiden oder Zähnen von 20 vorgesehen, die sich in der Teilung (Umfangswinkel) jeweils unterscheiden. Die Fig. 3 zeigt beispielhaft unterschiedliche Winkel. Es ergibt sich, dass keine Schneide der anderen gleicht. Durch die unterschiedlichen Teilungen ergeben sich unterschiedliche Schneidengeometrien sowie unterschiedliche Schneidentiefen, so wie dies oben stehend erläutert wurde.

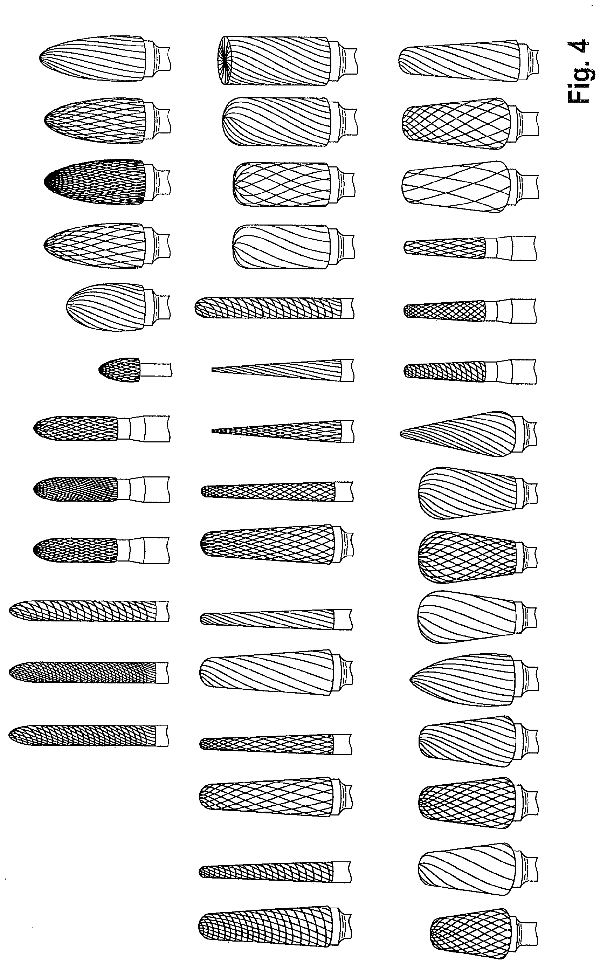

Die Fig. 4 zeigt in schematischer Seitenansicht eine Vielzahl möglicher Ausgestaltungen eines Kopfes eines erfindungsgemäßen Instruments. Diese Köpfe können beliebige Kopfformen aufweisen, beispielsweise zylindrisch oder konisch, jeweils mit stirnseitiger Abrundung oder Abplattung sowie weitere Formen, beispielsweise eine Birnenform, eine Flammenform, eine Knospenform oder ähnliches.

### Bezugszeichenliste:

- 1: Schaft
- 2: Kopf
- 3: Schneide
- 4: Drehachse
- 5: Drehkreis
- 6: Querschneide
- 7: Nebenschneide

## Patentansprüche

1. Dentalfräser, mit einem Schaft (1) und einem an dem Schaft (1) befestigten, mit mehreren auf einem gemeinsamen Drehkreis angeordneten Schneiden (3) versehenen Kopf (2), wobei die Schneiden (3) um den gesamten Umfang des Kopfes (2), bezogen auf eine zentrische Drehachse (4) des Instruments, verteilt angeordnet sind, **dadurch gekennzeichnet, dass** in einer zur Drehachse (4) senkrechten Radialebene jede Schneide (3) eine zu den anderen Schneiden (3) unterschiedliche Geometrie aufweist, wobei in der Radialebene jede Schneide (3) eine andere Teilung und/oder eine andere Schneidentiefe und/oder einen anderen Keilwinkel und/oder einen anderen Spanwinkel und/oder einen anderen Freiwinkel aufweist.

2. Dentalfräser nach Anspruch 1, **dadurch gekennzeichnet, dass** die Schneiden (3) alle auf einem gemeinsamen Drehkreis (5), welcher seinen Mittelpunkt auf der Drehachse (4) hat, angeordnet sind.

3. Dentalfräser nach einem der Ansprüche 1 oder 2, **dadurch gekennzeichnet, dass** die Schneiden (3) über die gesamte axiale Länge des Kopfes (2) ausgebildet sind.

4. Dentalfräser nach einem der Ansprüche 1 bis 3, **dadurch gekennzeichnet, dass** die Schneiden (3) in zwei oder mehr Gruppen angeordnet sind.

5. Dentalfräser nach einem der Ansprüche 1 bis 4, **dadurch gekennzeichnet, dass** die Schneiden (3) stirnseitig am Kopf zur Drehachse (4) verlaufend ausgebildet sind.

6. Dentalfräser nach einem der Ansprüche 1 bis 4, **dadurch gekennzeichnet, dass** die Schneiden (3) stirnseitig zur Ausbildung zumindest einer Querschneide (6) angeordnet sind.

7. Dentalfräser nach einem der Ansprüche 1 bis 6, **dadurch gekennzeichnet, dass** die Schneiden (3) gerade oder gedrallt ausgebildet sind.

## Claims

1. Dental cutter, having a shaft (1) and a head (2) that is attached to the shaft (1) and is provided with multiple cutting edges (3) that are arranged on a common turning circle, wherein the cutting edges (3) are arranged so as to be distributed about the entire circumference of the head (2) with respect to a centric rotational axis (4) of the instrument, **characterized in that** each cutting edge (3) has a geometry that differs from the other cutting edges (3) in a radial plane that is perpendicular to the rotational axis (4), wherein each cutting edge (3) has a different division and/or a different cutting edge depth and/or a different wedge angle and/or a different rake angle and/or a different clearance angle in the radial plane.

2. Dental cutter according to claim 1, **characterized in that** the cutting edges (3) are all arranged on a common turning circle (5) that has its center point on the rotational axis (4).

3. Dental cutter according to one of the claims 1 or 2, **characterized in that** the cutting edges (3) are embodied across the entire axial length of the head (2).

4. Dental cutter according to one of the claims 1 to 3, **characterized in that** the cutting edges (3) are arranged in two or more groups.

5. Dental cutter according to one of the claims 1 to 4, **characterized in that** the cutting edges (3) are embodied so as to extend at the rotational axis (4) at the head on the front-face side.

6. Dental cutter according to one of the claims 1 to 4, **characterized in that** the cutting edges (3) are arranged on the front-face side for forming at least one chisel edge (6).

7. Dental cutter according to one of the claims 1 to 6, **characterized in that** the cutting edges (3) are embodied in a straight or twisted manner.

## Revendications

1. Fraise dentaire, comprenant une tige (1) et une tête (2) fixée au niveau de la tige (1), pourvue de plusieurs tranchants (3) agencés sur un cercle rotatif commun, dans laquelle les tranchants (3) sont agencés de manière répartie autour de l'ensemble du pourtour de la tête (2) par rapport à un axe de rotation (4) central de l'instrument, **caractérisée en ce que** chaque tranchant (3) présente, dans un plan radial perpendiculaire par rapport à l'axe de rotation (4), une géométrie différente par rapport aux autres tranchants (3), dans laquelle chaque tranchant (3) présente, dans le plan radial, un autre pas et/ou une autre profondeur de coupe et/ou un autre angle de taillant et/ou un autre angle d'enlèvement de copeaux et/ou un autre angle libre.

2. Fraise dentaire selon la revendication 1, **caractérisée en ce que** les tranchants (3) sont tous agencés sur un cercle rotatif (5) commun, qui a son point central sur l'axe de rotation (4).

3. Fraise dentaire selon l'une quelconque des revendications 1 ou 2, **caractérisée en ce que** les tranchants (3) sont réalisés sur l'ensemble de la longueur axiale de la tête (2).

4. Fraise dentaire selon l'une quelconque des revendications 1 à 3, **caractérisée en ce que** les tranchants (3) sont agencés en deux groupes ou plus.

5. Fraise dentaire selon l'une quelconque des revendications 1 à 4, **caractérisée en ce que** les tranchants (3) sont réalisés côté frontal au niveau de la tête de manière à s'étendre en direction de l'axe de rotation (4).

6. Fraise dentaire selon l'une quelconque des revendications 1 à 4, **caractérisée en ce que** les tranchants (3) sont agencés côté frontal afin de réaliser au moins un tranchant transversal (6).

7. Fraise dentaire selon l'une quelconque des revendications 1 à 6, **caractérisée en ce que** les tranchants (3) sont réalisés de manière droite ou de manière vrillée.
